# EUROPEAN PATENT SPECIFICATION

(11) **EP 2 177 174 B1**
(45) Date of publication and mention of the grant of the patent: **24.07.2013**
(21) Application number: 10150323.3
(22) Date of filing: 17.05.2007
(51) Int. Cl.: A61B 19/00

(54) **Robotic instrument system**
Roboterinstrumentensystem
Système d'instrument robotisé

(30) Priority: 17.05.2006 US 801355 P; 17.05.2006 US 801546 P; 18.05.2006 US 801945 P
(43) Date of publication of application: 21.04.2010
(62) Divisional of application: 07795082.2
(73) Proprietor: Hansen Medical, Inc., Mountain View, CA 94043 (US)
(72) Inventor: Barbagli, Federico, San Francisco, CA 94131 (US)
(74) Representative: Lecomte, Didier

(56) References cited:
- WO-A1-2007/005976
- US-A- 6 102 850
- US-A1- 2006 084 945

## Description

### FIELD OF INVENTION

The invention relates generally to robotically-controlled medical instrument systems, and more particularly to robotically-controlled flexible instrument systems configured for use in minimally-invasive medical intervention and diagnosis.

### BACKGROUND

Robotic instrument (e.g., catheter) systems and devices are well suited for use in performing minimally invasive medical procedures, as opposed to conventional techniques wherein the patient's body cavity is open to permit the surgeon's hands access to internal organs. For example, there is a need for a highly controllable yet minimally sized system to facilitate imaging, diagnosis, and treatment of tissues which may lie deep within a patient, and which may be accessed via naturally-occurring pathways such as blood vessels or the gastrointestinal tract, or small surgically-created pathways.

Patent document WO 2007/005976 A1 which is prior art for novelty only discloses a robotic instrument system comprising a controller including a master input device and an operator control console; and an instrument driver in communication with the controller, the instrument driver comprising a guide instrument interface including two or more guide instrument drive elements responsive to control signals generated, at least in part, by the master input device for manipulating a guide instrument operatively coupled to the instrument interface, and a sheath instrument interface including two or more sheath instrument drive elements responsive to control signals generated, at least in part, by the master input device for manipulating a sheath instrument operatively coupled to the instrument insterface, wherein the sheath instrument drive elements are controlled independently of the guide instrument control elements.

Patent document US 2006/084945 A1 discloses a robotic instrument system with a guide instrument interface and a sheath instrument interface where the sheath instrument interface comprises a single drive element.

### SUMMARY OF THE INVENTION

In accordance with the invention, a robotic instrument system is provided in accordance with claims 1 and 2. Embodiments of the invention are disclosed in the dependent claims 3 to 12.

### BRIEF DESCRIPTION OF THE DRAWINGS

The drawings illustrate the design and utility of illustrated embodiments of the invention, in which similar elements are referred to by common reference numerals, and in which:
Fig. 1 illustrates a robotic catheter system in accordance with one embodiment;
Fig. 2 illustrates a robotic catheter system operator workstation in accordance with one embodiment;
Fig. 3 illustrates a perspective view of a master input device in accordance with one embodiment;
Fig. 4A illustrates a side view of a master input device in accordance with another embodiment;
Fig. 4B illustrates a magnified partial side view of the master input device embodiment of Fig. 4A;
Fig. 4C illustrates an exploded partial perspective view of the master input device embodiment of Fig. 4A;
Fig. 4D illustrates a partial perspective view of the master input device embodiment of Fig. 4A;
Fig. 4E illustrates a magnified partial rear view of the master input device embodiment of Fig. 4A;
Fig. 4F illustrates a magnified partial perspective view of the master input device embodiment of Fig. 4A;

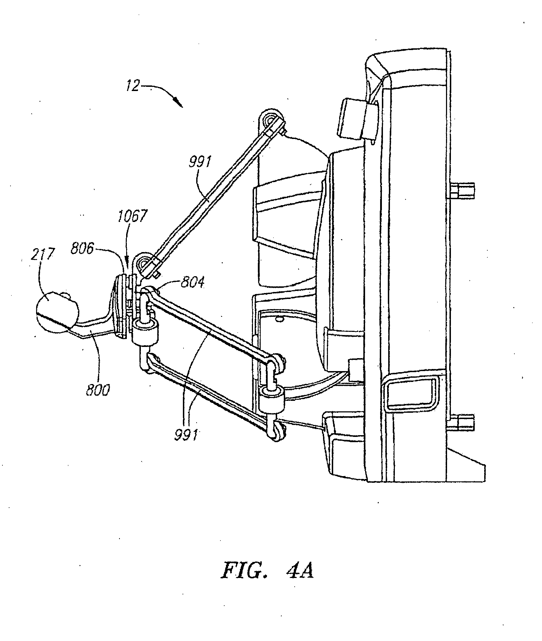

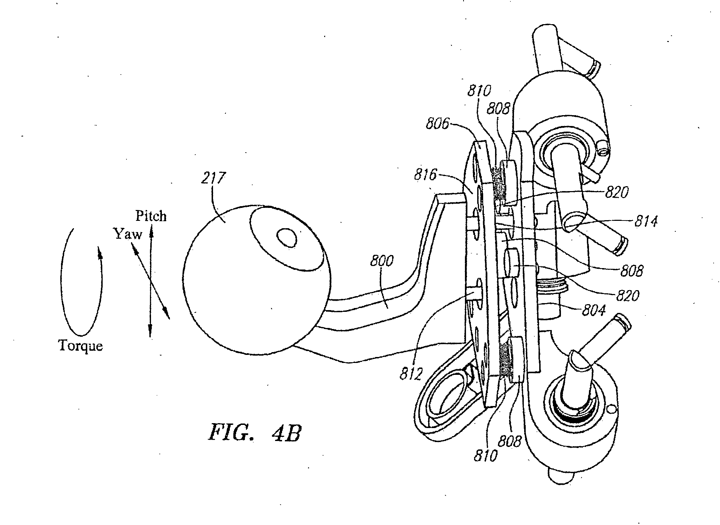

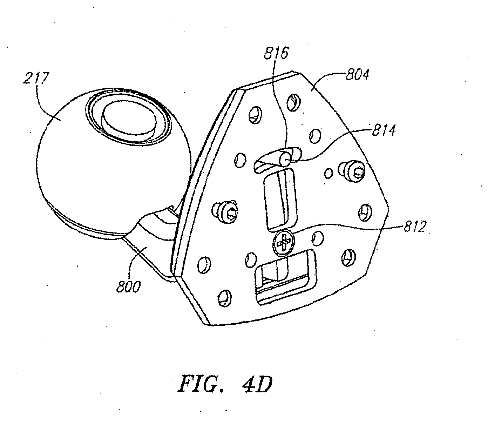

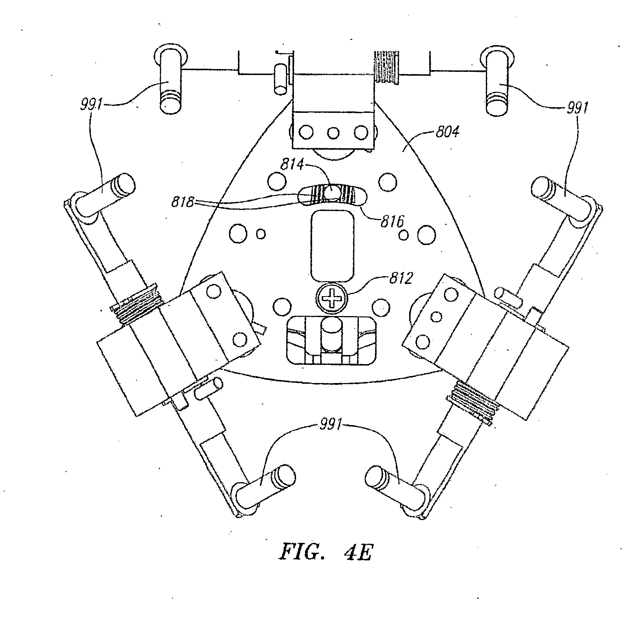

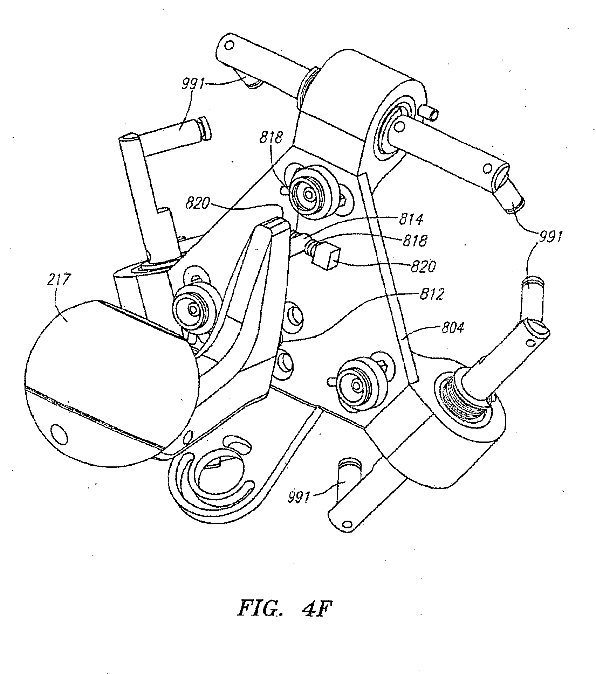

Fig. 4B illustrates a magnified partial side view of the master input device embodiment of Fig. 4A;

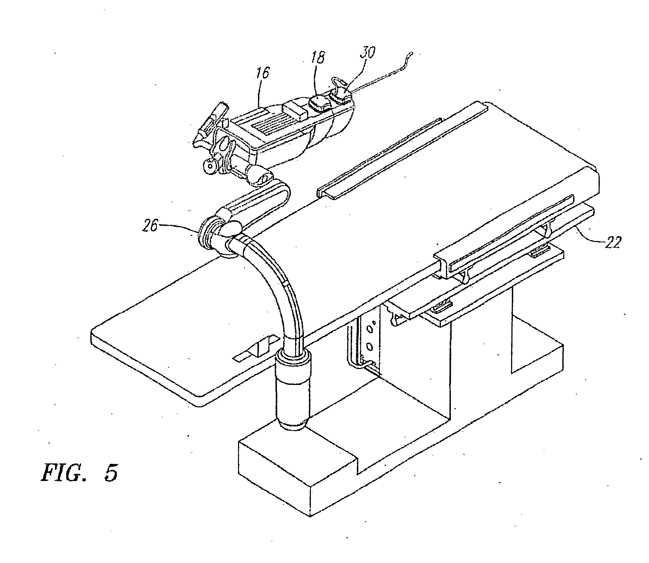

Fig. 5 illustrates a robotic catheter system in accordance with one embodiment;

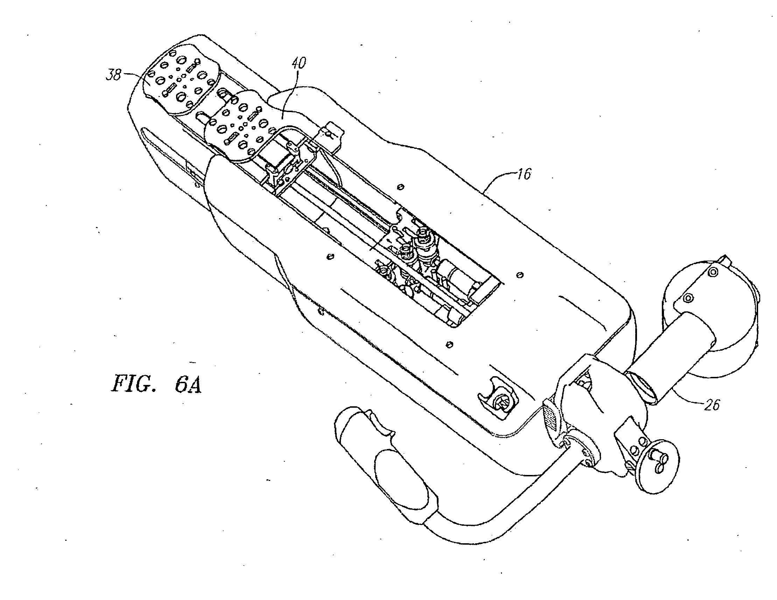

Fig. 6A illustrates a robotic instrument driver in accordance with one embodiment;

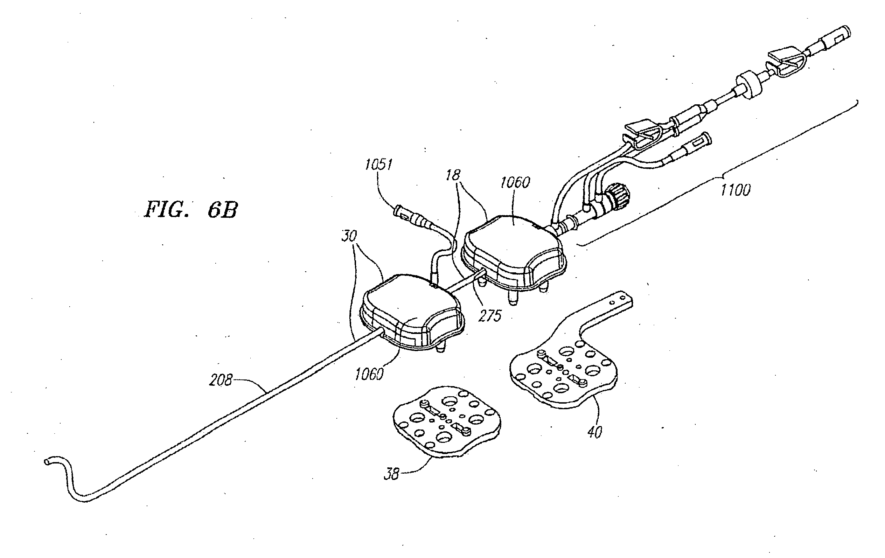

Fig. 6B illustrates an perspective view of an instrument set and instrument interface members in accordance with one embodiment;

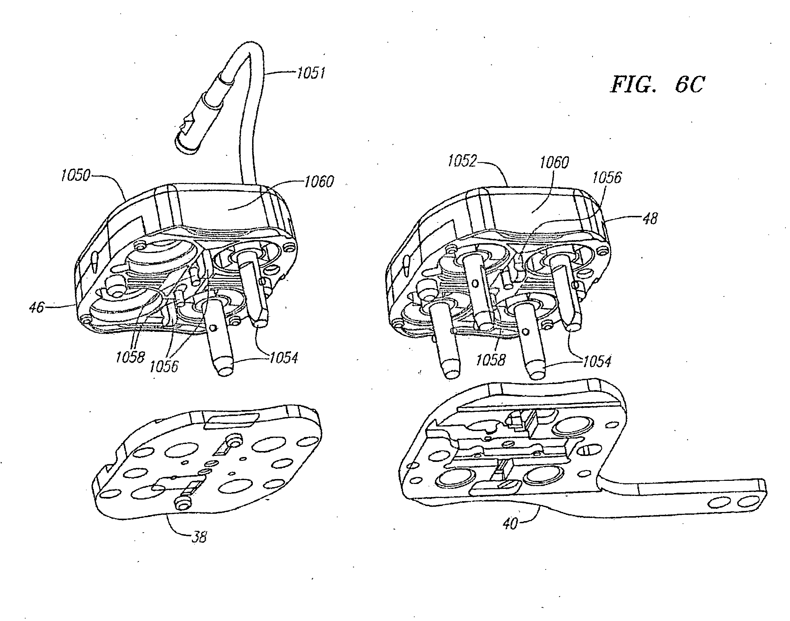

Fig. 6C illustrates a partial perspective view of an instrument set and instrument interface members in accordance with one embodiment;

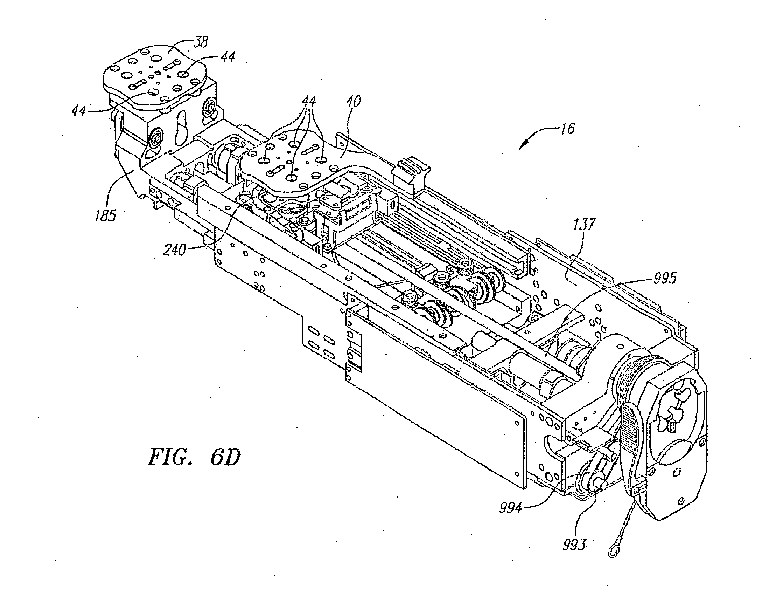

Fig. 6D illustrates a partial perspective view of an instrument driver in accordance with one embodiment;

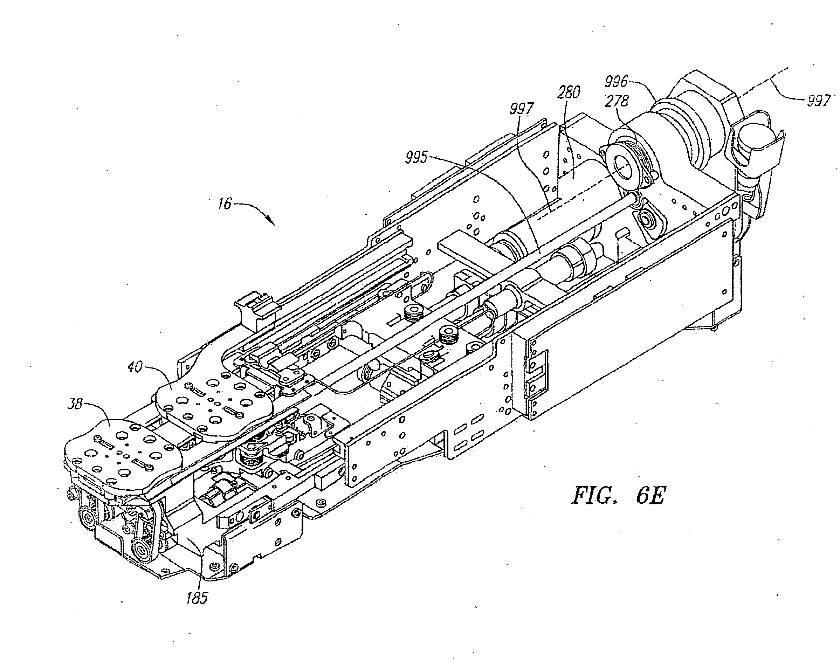

Fig. 6E illustrates a partial perspective view of an instrument driver in accordance with one embodiment;

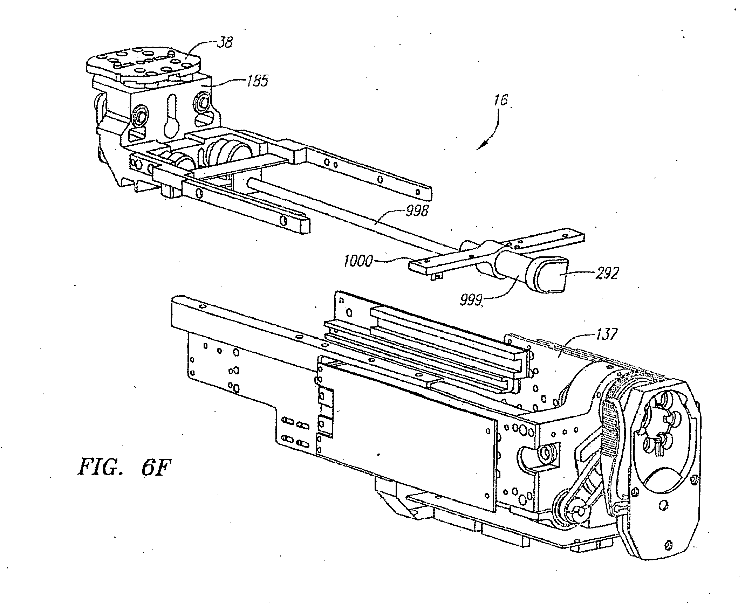

Fig. 6F illustrates a partial perspective view of an instrument driver in accordance with one embodiment;

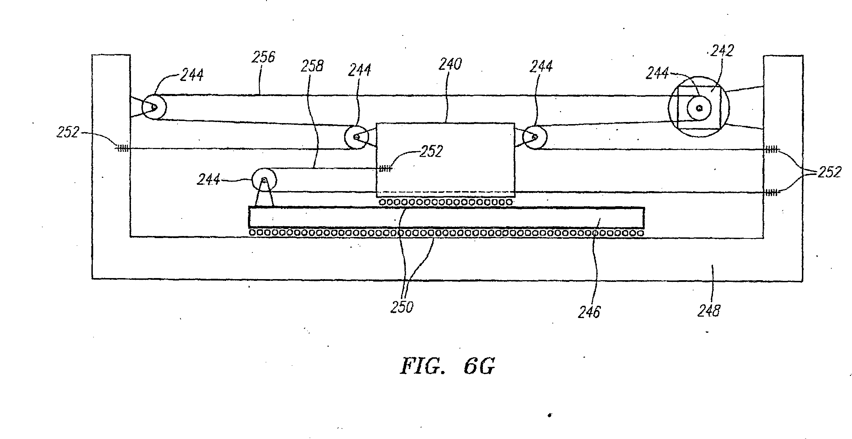

Fig. 6G illustrates a diagrammatic representation of an instrument carriage insertion configuration in accordance with one embodiment;

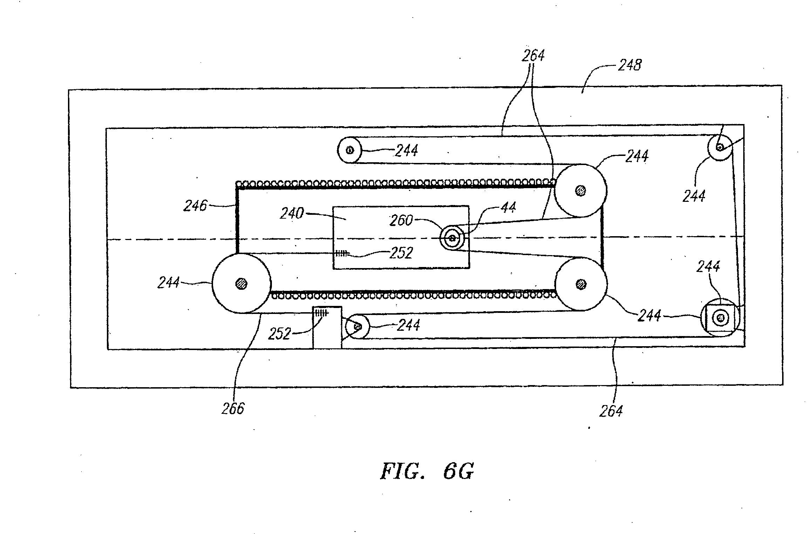

Fig. 6H illustrates a diagrammatic representation of an instrument interface socket and instrument carriage configuration in accordance with one embodiment;

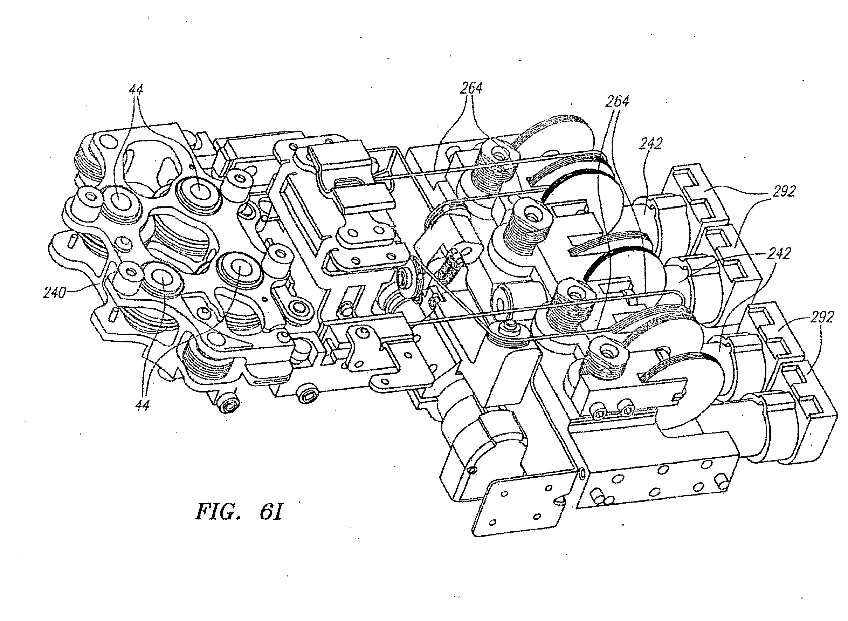

Fig. 6I illustrates a partial perspective view of an instrument driver in accordance with one embodiment;

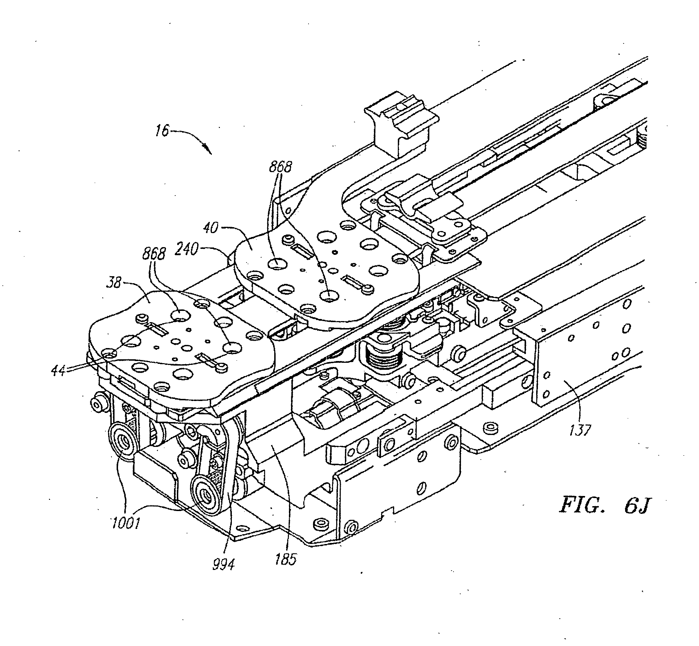

Fig. 6J illustrates a partial perspective view of an instrument driver in accordance with one embodiment;

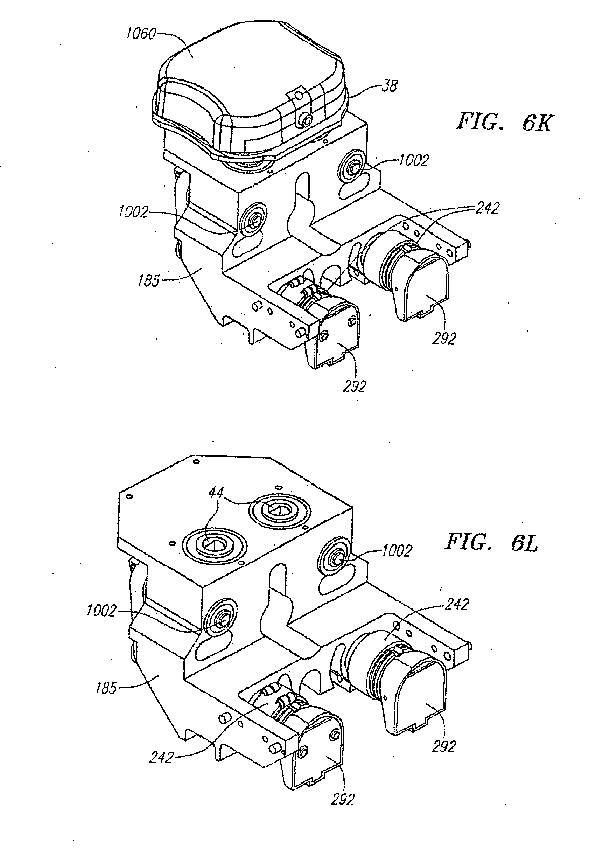

Fig. 6K illustrates a partial perspective view of an instrument driver in accordance with one embodiment;
Fig. 6L illustrates a partial perspective view of an instrument driver in accordance with one embodiment;

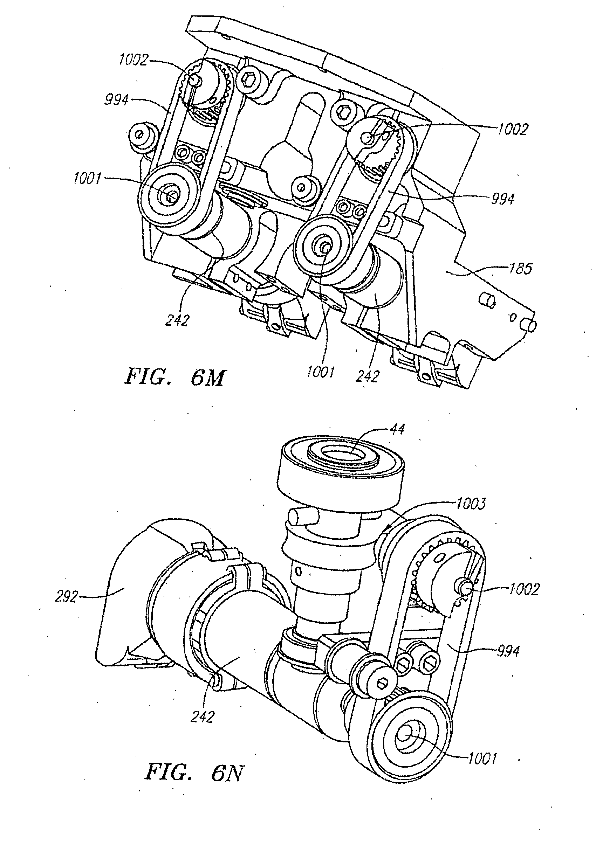

Fig. 6M illustrates a partial perspective view of an instrument driver in accordance with one embodiment;
Fig. 6N illustrates a partial perspective view of an instrument driver in accordance with one embodiment;

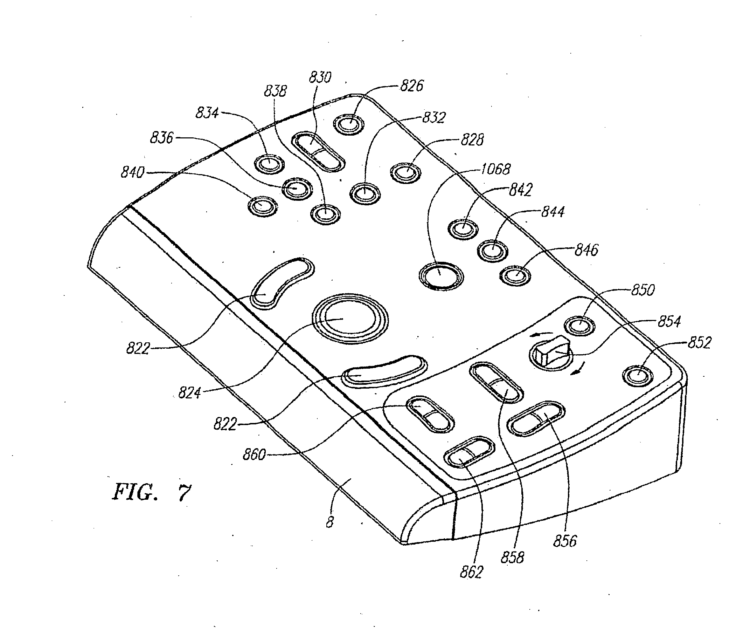

Fig. 7 illustrates a perspective view of an operator control console in accordance with one embodiment;

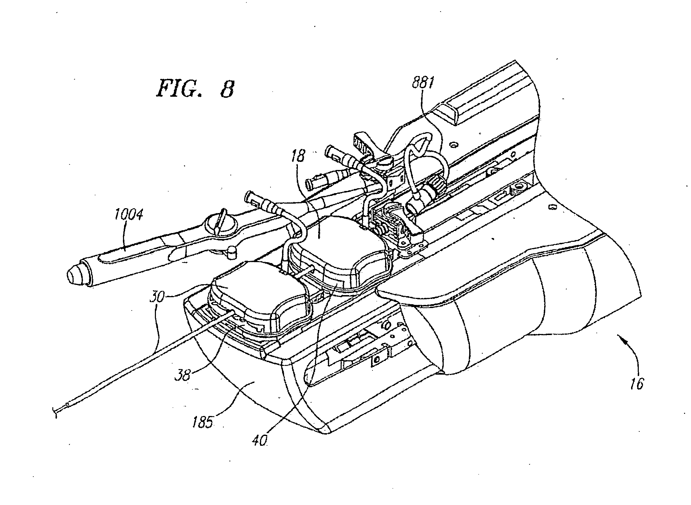

Fig. 8 illustrates a partial perspective view of an instrument driver and instruments in accordance with one embodiment;

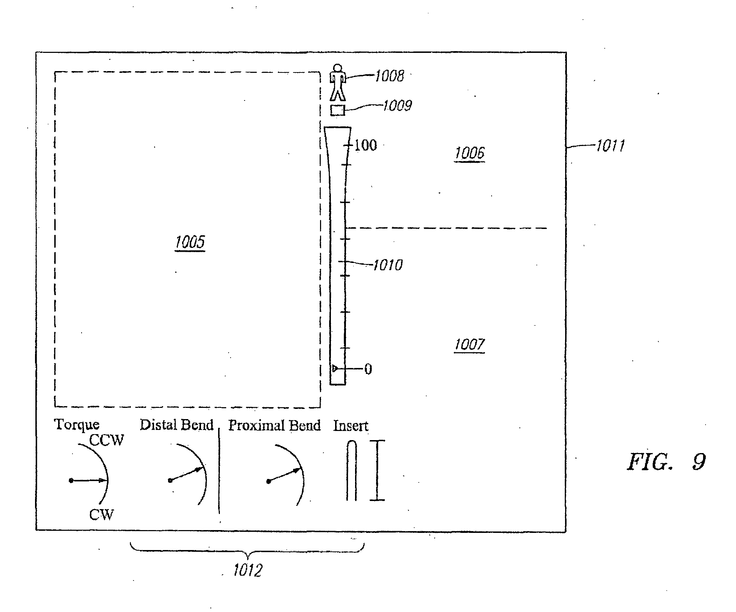

Fig. 9 illustrates a user interface displayed in accordance with one embodiment;

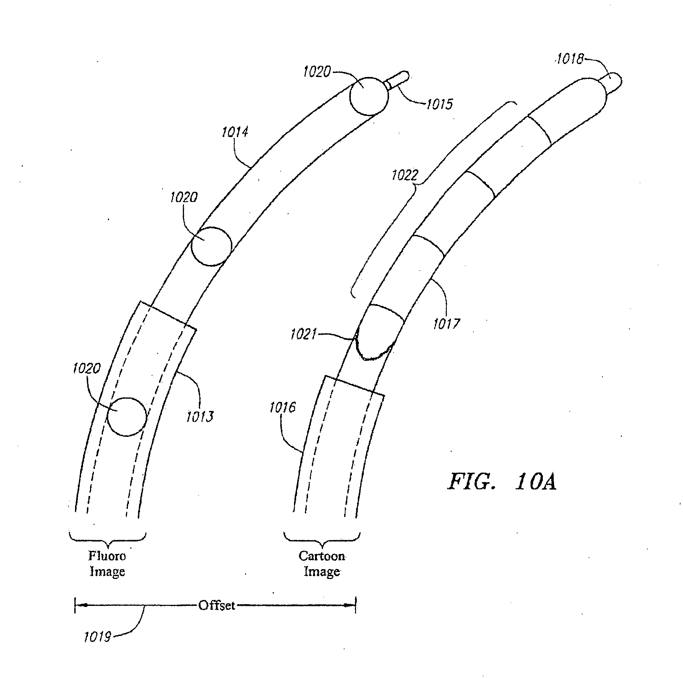

Fig. 10A illustrates a user interface displayed in accordance with one embodiment;

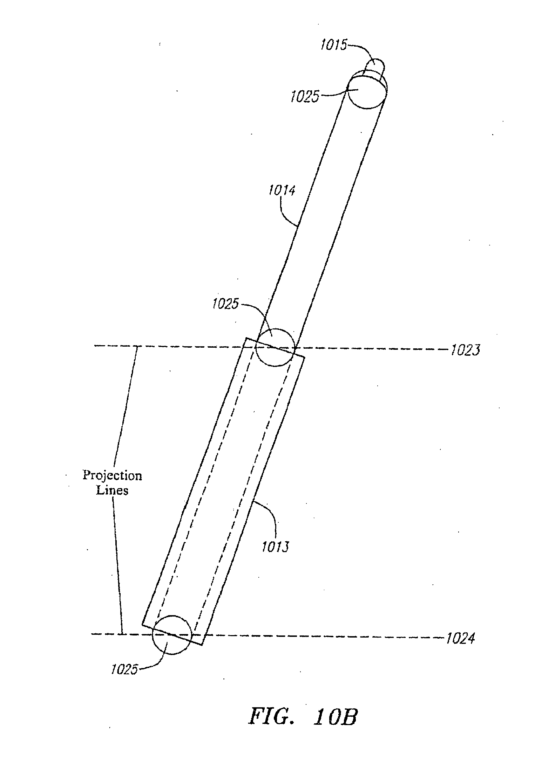

Fig. 10B illustrates a user interface displayed in accordance with one embodiment;

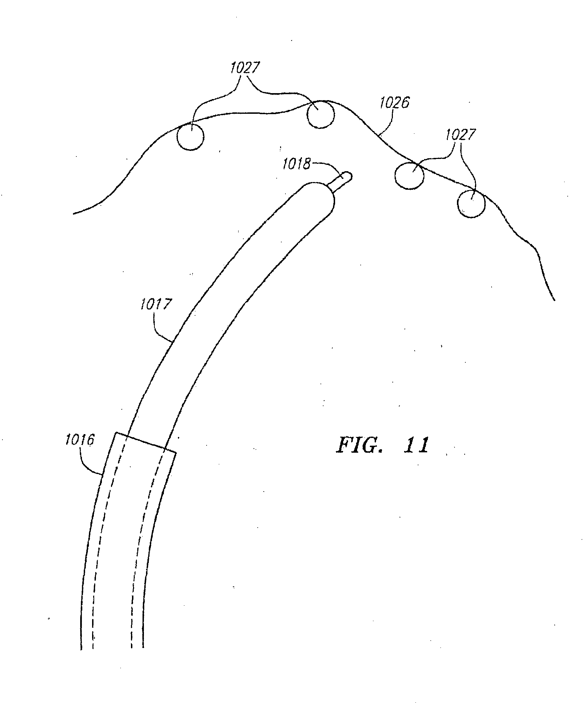

Fig. 11 illustrates a user interface displayed in accordance with one embodiment;

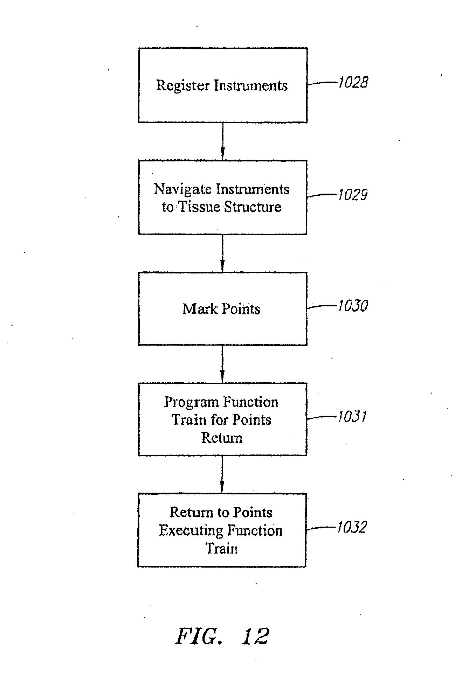

Fig. 12 illustrates an operational configuration in accordance with one embodiment;

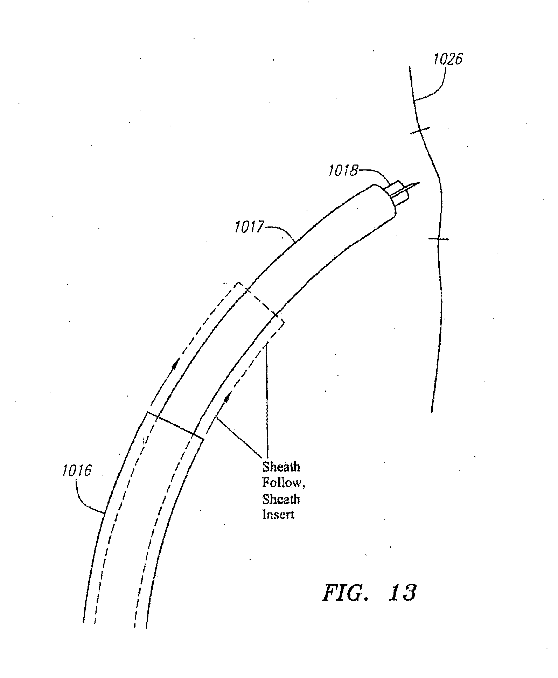

Fig. 13 illustrates a user interface displayed in accordance with one embodiment;

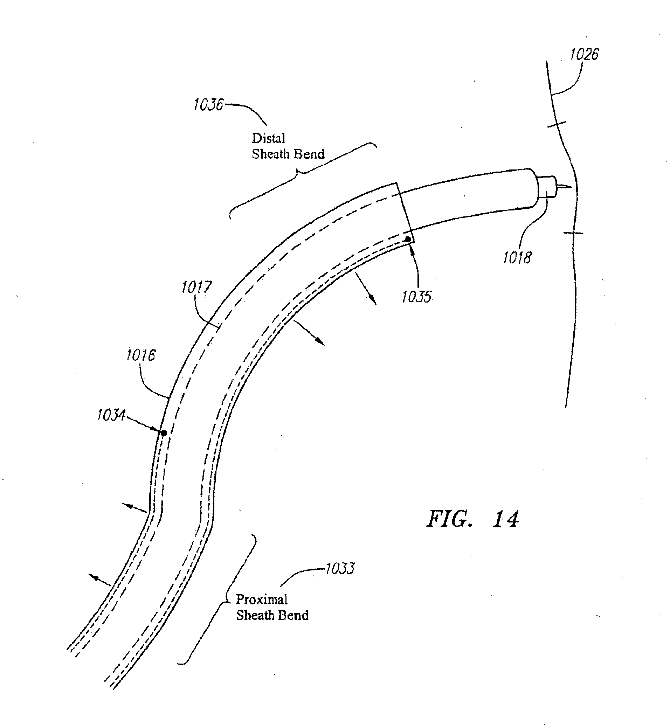

Fig. 14 illustrates a user interface displayed in accordance with one embodiment;

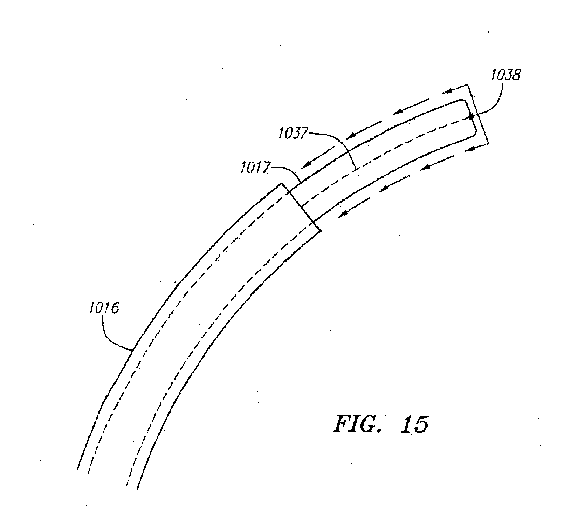

Fig. 15 illustrates a user interface displayed in accordance with one embodiment;

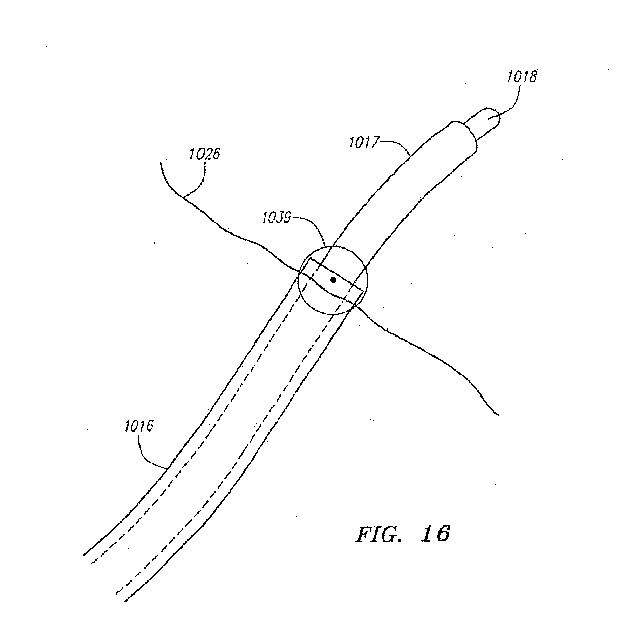

Fig. 16 illustrates a user interface displayed in accordance with one embodiment;

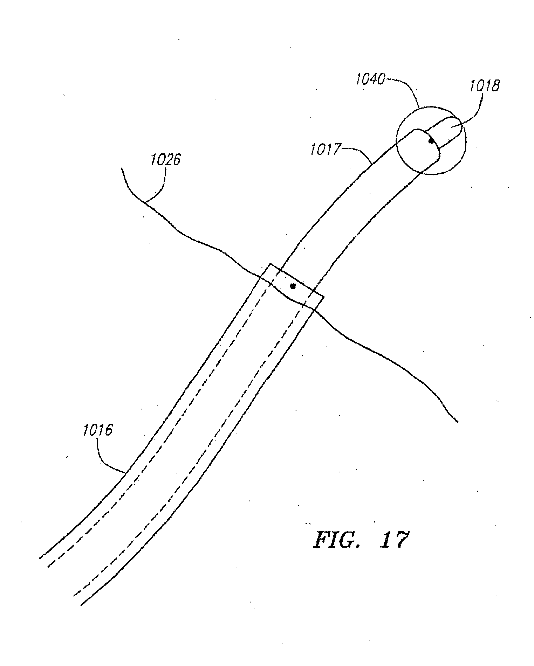

Fig. 17 illustrates a user interface displayed in accordance with one embodiment;

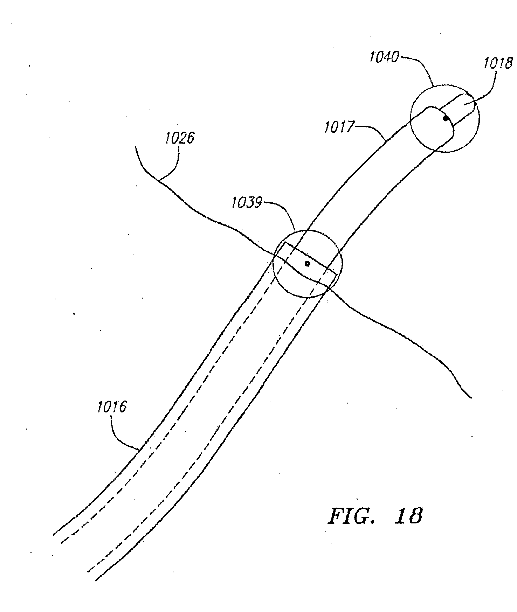

Fig. 18 illustrates a user interface displayed in accordance with one embodiment;

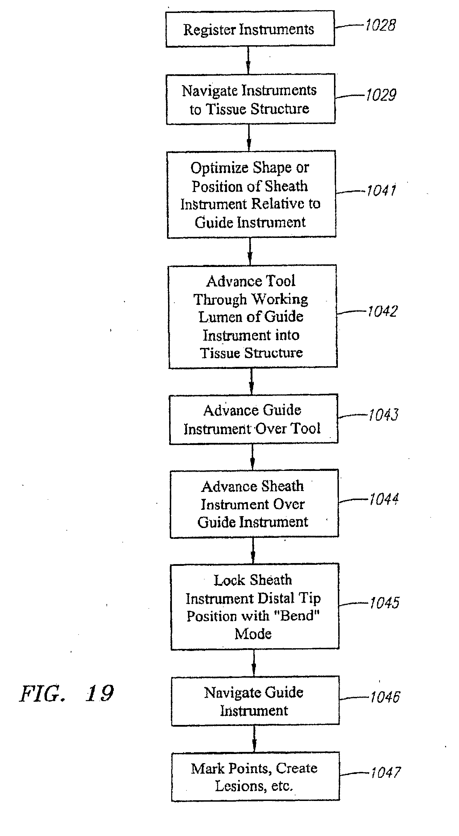

Fig. 19 illustrates an operational configuration in accordance with one embodiment;

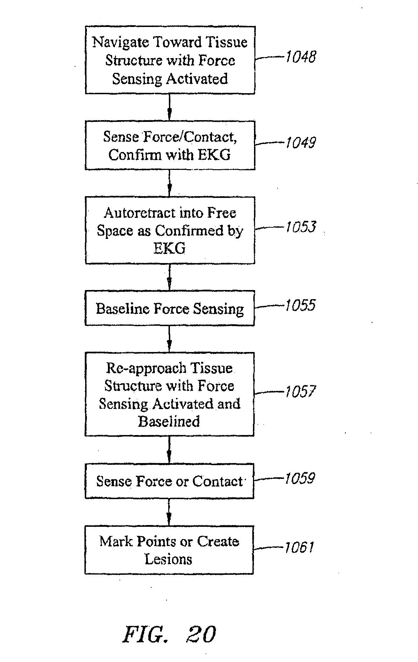

Fig. 20 illustrates an operational configuration in accordance with one embodiment;

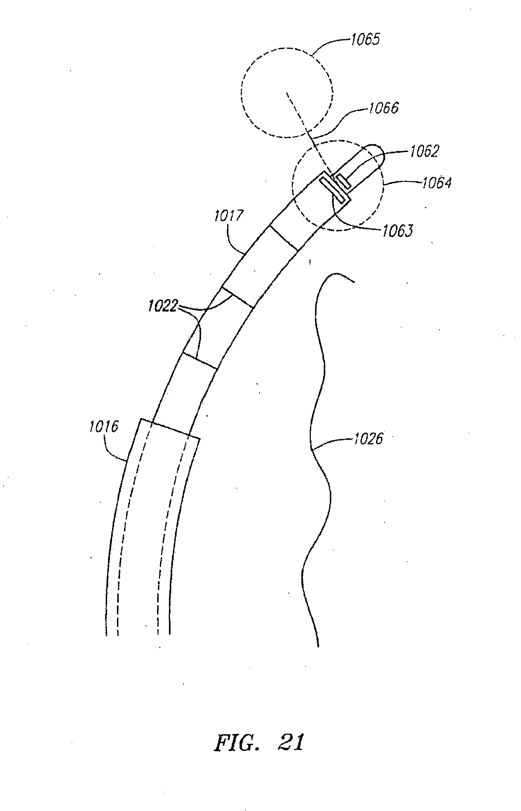

Fig. 21 illustrates a user interface displayed in accordance with one embodiment.

### DETAILED DESCRIPTION OF THE ILLUSTRATED EMBODIMENTS

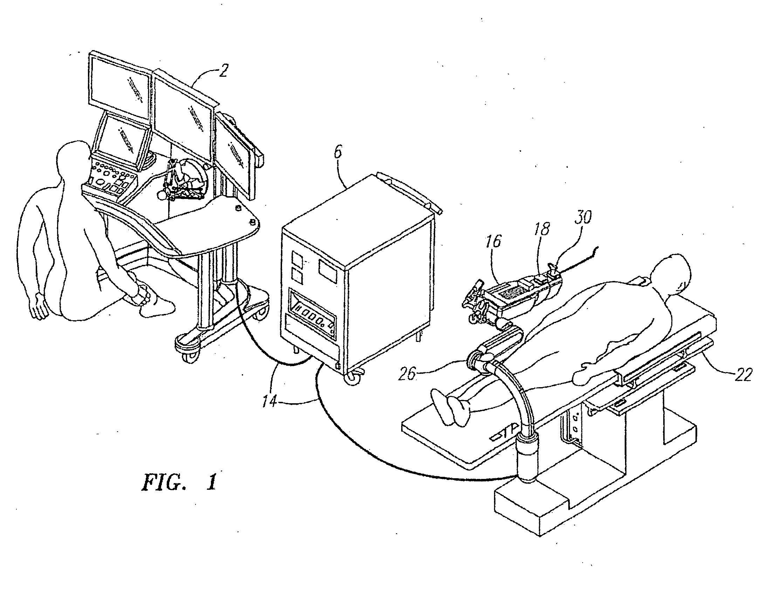

Referring to Figure 1, a robotic catheter system is depicted comprising an operator workstation (2), a movable computing and control system (6), a robotic instrument driver (16), a movable support assembly (26), which is removably mounted to an operating table (22), and an instrument set comprising a guide instrument (18) coaxially positioned through the working lumen of a sheath instrument (30), the guide instrument defining a working lumen configured to receive tools of various configurations (not shown in Figure 1) for interventional and diagnostic medical procedures.

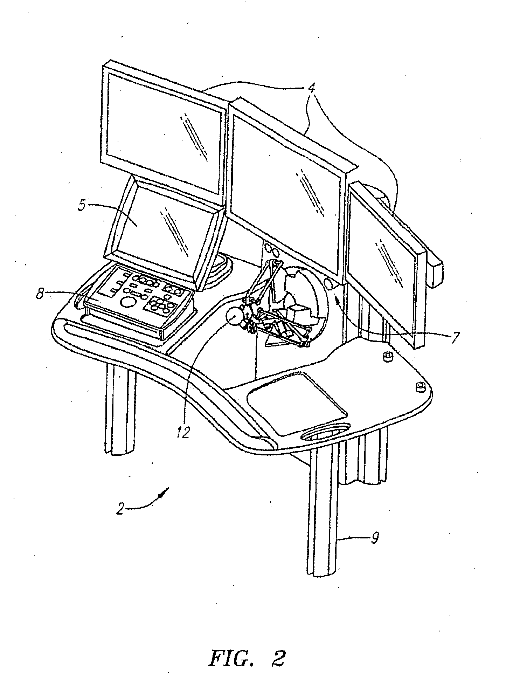

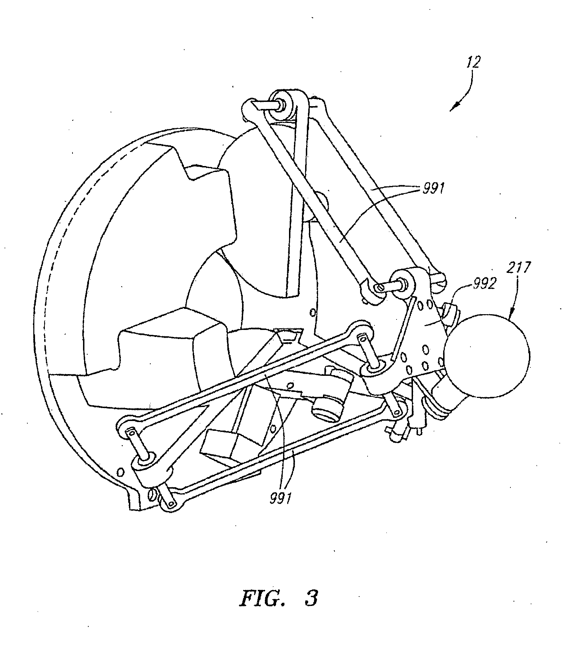

Referring to Figure 2, a closer view of an embodiment of an operator workstation (2) is depicted, comprising a master input device (12), an operator control console (8), a touchscreen interface (5), a device disabling switch (7), and several displays (4). Referring to Figure 3, a closer isometric view of one embodiment of a suitable master input device (12) is depicted having a substantially spherical operator interface (217) coupled to a master interface link assembly (991) by a master interface link assembly member (992) which is preferably fixedly attached to the operator interface (217). Suitable master input devices are available from manufacturers such as Sensible Devices Corporation under the trade name "Phantom™", or Force Dimension under the trade name "Omega™". Referring to Figures 4A-4F, another embodiment of a suitable master input device (12) is depicted.

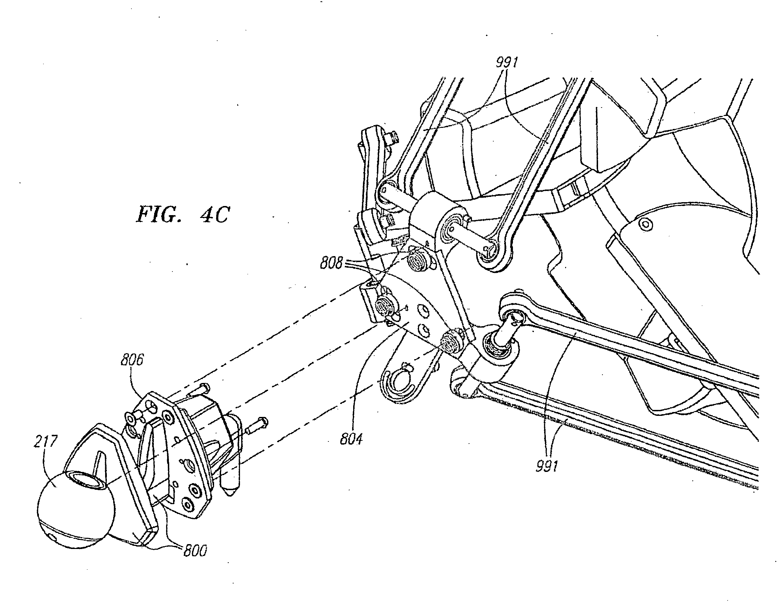

Referring to Figure 4A, a master input device (12) configuration is depicted having a three degree of freedom ("DOF") interface interposed between the operator interface (217) and the master input device link assembly member (804). Referring to Figure 4A, the operator interface (217) is coupled to the master interface link assembly (991) by two interface mounting members (804, 806), between which several load sensors are interfaced. Referring to Figure 4B, a close-up partial side view of the master input device is depicted to show three load cells (808) and three adjacent springs (810) interfaced between the master input device link assembly member (804) and the operator interface mounting member (806). Also depicted is a shaft (814) which is coupled to the operator interface mount (800), passed through an arcuate slot (816) formed in the operator interface mounting member (806), and positioned between two smaller load cells (820). The operator interface mount (800) is coupled to the operator interface mounting member (806) with a pivoting mounting fastener (812) which is configured to allow the operator interface mount (800) to rotate about the pivoting mounting fastener (812) approximately in the plane of the operator interface mounting member (806) to cause the shaft (814) to move through the slot (816) and cause the small load cells (820) to sense torque upon the operator interface (217), as illustrated in Figure 4B. The load cells (808) sensing forces between the operator interface mounting member (806) and the master input device link assembly member (804) may be utilized in concert to sense pitch and yaw loads at the operator interface (217). Thus, three degrees of freedom may be sensed with this variation of the master input device. In one embodiment, the torque degree of freedom may be interpreted as an attempt by the operator to actuate an electromechanical roll an instrument driver and associated elongate instruments. The pitch and yaw degrees of freedom may be assigned to various variables or degrees of freedom of the pertinent instrument driver and/or instrument. For example, in one embodiment, the pitch degree of freedom may be assigned to a sheath insertion actuation degree of freedom such that a positive pitch (beyond a selected minimum threshold load to prevent the pitch DOF from being accidentally triggered as the operator interface 217 is being gently moved about in 3-D space to steer the tip of the guide instrument, for example) may be interpreted as a signal that the operator wishes the sheath instrument to insert distally toward the distal tip of the guide instrument; similarly negative pitch can be assigned to negative insert - to pull the sheath instrument proximally. Figures 4C-4F show additional partial views of this variation of the master input device (12) to more fully illustrate the relative positions of the operator interface mount (800), master input device frameset (802), master input device link assembly member (804), operator interface mount member (806), load cells (808, 820), springs (810, 818), pivot mount interface (812), shaft (814), and arcuate slot (816) formed in the operator interface mounting member (806).

Referring to Figure 5, an isometric view of one embodiment of an instrument driver (16) coupled to an operating table (22) by a movable support assembly (26) is depicted. Guide (18) and sheath (30) instruments are depicted removably coupled to the instrument driver, as they preferably would be during a medical procedure utilizing the subject robotic catheter system.

Referring to Figure 6A, one embodiment of an instrument driver is depicted is close up isometric view having a sheath instrument interface surface (38) positioned distally of a guide instrument interface surface (40). The proximal portion of the instrument driver (16) is coupled to the distal portion of the movable support assembly (26).

Referring to Figure 6B, one embodiment of an instrument set, comprising a guide instrument (18), a sheath instrument (30), and associated guide and sheath base covers (1060), sheath purge tube (1051), and Touhy assembly (1100), is depicted adjacent a sheath instrument interface surface (38) and guide instrument interface surface (40) to illustrate how the instrument set (30, 18) is configured to interface with the interface surfaces (38, 40). Referring to Figure 6C, a different partial isometric view illustrates how the underside of the sheath instrument base (46) and guide instrument base (48), and associated instrument interface members (1054) are configured to geometrically interface with the sheath instrument interface surface (38) and guide instrument interface surface (40). Each of the guide instrument base (48) and sheath instrument base (46) embodiments depicted also has at least one pin (1058) extending toward the pertinent interface surface (38, 40) to facilitate detection of interfacing therewith, by virtue of a compressive electronic switch (not shown) associated with each pin (1058). Further, each of the guide instrument base (48) and sheath instrument base (46) embodiments depicted also has at least one coupling member (1056) extending toward and engagably coupling with the pertinent interface surface (38, 40) to facilitate removable coupling with geometric aspects of such surface.

Referring to Figures 6D-6F, partial perspective views of one embodiment of an instrument driver (16) is depicted to illustrate that the sheath instrument driver block (185), to which the sheath instrument interface (38) is coupled, and the guide carriage (240), to which the guide instrument interface (40) is coupled, may be inserted along the longitudinal axis is the instrument driver (or along the longitudinal axis of each of the lead screws (995, 998), for that matter), independently of each other, by virtue of two independently-actuated lead screw (995, 998) mechanisms in such embodiment. Referring to Figure 6D, in one embodiment, the instrument driver (16) is configured to independently insert the sheath instrument interface (38) and the guide instrument interface (40), to cause independent relative insertion (relative to each other, and relative to the main frame (137) of the instrument driver) of a detachably coupled instrument set comprising a sheath instrument and guide instrument. The guide and sheath instruments are configured to have driveable instrument interface members (1054), as depicted in Figure 6C, engageable to driven interface sockets (44) underlying the guide and sheath interfaces (40, 38).

In the embodiments depicted in Figures 6C and 6D, a sheath instrument (30) having two driveable interface members (1054), and a guide instrument having four driveable interface members (1054) are utilized to provide two steering actuations of the sheath instrument (30) and four steering actuations of the guide instrument (18); in the preferred embodiment, such steering actuations are utilized as follows: one sheath steering actuation engages a distally-terminated tension element, such as a pullwire threaded through a wall of the sheath instrument, to steer the sheath instrument in a first direction; one sheath steering actuation engages a more-proximally-terminated tension element, such as a pullwire threaded through a wall of the sheath instrument, to cause a bending of the sheath instrument in a direction substantially opposite of the first direction at a location along the sheath instrument proximal to the proximal tension element termination point; each of the four guide steering actuations engages a distally-terminated tension element, such as a pullwire threaded through a wall of the guide instrument, to cause controllable omnidirectional steering of the guide instrument by virtue of combined tensions within the four tension elements.

In the depicted embodiment, a guide insertion motor (not shown) coupled to a guide insertion motor interface (993), such as a cable capstan or belt pulley, transfers motion to the guide insertion lead screw (995) utilizing a belt (994). The guide insertion lead screw (995) is coupled to a lead screw interface, such as a gear, gear tooth, or rack, coupled to the guide carriage (240) structure configured to cause the guide carriage (240) to insert back and forth parallel to the axis of the lead screw (995), independent of the position of the sheath block (185) or the instrument driver frame (137).

Referring to Figure 6E, an embodiment similar to that depicted in Figure 6D is depicted from a different perspective, illustrating the guide insertion lead screw (995), and also the roll actuation motor (280), which is interfaced with a roll drive interface (996), such as a gear, to actuate roll of the entire instrument driver (16) about the rotational axis (997) of a low-friction rotational mounting interface (278), such as a bearing or bushing. When such roll actuation is engaged in a clockwise or counterclockwise direction, the entire instrument driver (16) is rotated about the rotational axis (997) of the rotational mounting interface (278), causing any sheath, guide, and/or other instruments which may be interfaced with the instrument driver (16) to also roll, or in conventional catheter terminology, to "torque" relative to the patient's tissue.

Referring to Figure 6F, a partial exploded view of an instrument driver (16) embodiment similar to that depicted in Figure 6E is depicted is shown to illustrate a leadscrew-based means of inserting the sheath mounting block (185) relative to the main instrument driver frame (137), independently of the aforementioned guide carriage (240) insertion configuration embodiment. As shown in Figure 6F, a sheath insertion lead screw (998) is positioned between a sheath insertion motor (999) and associated encoder (292), and the sheath mounting block (185). The sheath insertion lead screw (998) is coupled to a lead screw interface, such as a gear, gear tooth, or rack, coupled to the sheath block (185) structure configured to cause the guide carriage (240) to insert back and forth parallel to the axis of the lead screw (998), independent of the position of the guide carriage (240) or the instrument driver frame (137).

Referring to Figure 6G, an alternative embodiment for inserting either a sheath block (185) or guide carriage (240) is illustrated in diagrammatic form, such embodiment utilizing a system of cables, pulleys, motors, and movably interfaced platforms; such configurations are alternative embodiments to the aforementioned lead screw insertion configurations. As shown in the embodiment of Figure 6G, a carriage (240) is slidably mounted upon a platform (246), which is slidably mounted to a base structure (248). The slidable mounting (250) at these interfaces may be accomplished with high-precision linear bearings. The depicted system has two cables (256, 258) running through a plurality of pulleys (244) to accomplish motorized, synchronized relative motion of the carriage (240) and platform (246) along the slidable interfaces (250). As will be apparent to those skilled in the art, as the motor (242) pulls on the carriage displacement cable (256) with a tension force T, the carriage (240) feels a force of 2*T. Further, as the motor pulls the carriage displacement cable (256) by a displacement X, the carriage moves by X/2, and the platform moves by half that amount, or X/4, due to its "pulleyed" synchronization cable (258).

Referring to Figure 6H, an embodiment of a configuration for electromechanically actuating a rotational instrument interface socket (44) independently of insertion position (i.e., insertion position of the sheath block (185) or guide carriage (240)) is depicted in simplified diagrammatic form - depicting only one driven instrument interface socket (44) and associated cabling for simplicity of illustration purposes. The embodiment of Figure 6H configured to drive an instrument interface pulley (260) associated with an instrument interface socket (44) to produce both directions of rotation independently from the position of the carriage (240) or sheath block (185 - analogy not shown), to which it is coupled, along the linear pathway prescribed by the slidable interfaces (250). In summary, with a mechanical schema similar to that illustrated in Figure 6H, as the motor (242) pulls a deflection X in the instrument interface cable (264), the same deflection is seen directly at the instrument interface pulley (260), regardless of the position of the carriage (240) relative to the motor (242), due to the synchronizing cable (266) positioning and termination (252). Such a configuration may be multiplied by two, in the case of the aforementioned two-actuation sheath instrument steering, and by four, in the case of the aforementioned four-actuation guide instrument steering. Indeed, referring to Figure 6I, such an embodiment of a four independently actuated guide instrument interface socket (44) carriage (240) is depicted, showing the cabling (264) and associated four motors (242) and encoders (292).

Referring to Figures 6J-6N, additional partial isometric views of an instrument driver (16) embodiment are depicted to illustrate one embodiment for independently actuating two sheath instrument interface sockets (44). Once again, the depicted instrument driver embodiment is configured to independently rotatably actuate two sheath instrument interface members (1054 in Figure 6C) and four guide instrument interface members (1054 in Figure 6C), while also independently inserting the sheath block (185) and guide carriage (240) relative to each other and the instrument driver frame (137). Holes (868) are defined within the sheath and guide instrument interfaces (38, 40) to accommodate passage of such instrument interface members (1054 in Figure 6C) into the underlying instrument interface sockets (44) for rotatable actuation. Referring to Figure 6J, two motor-driven interfaces (1001), such as shafts and/or pulleys or gears, may be independently rotated by motors (see Figures 6K-6N for motors (241)) which are fixedly coupled to the sheath block (185) in the depicted embodiment. Such interfaces (1001) are mechanically interfaced with secondary interfaces (1002), such as shafts and/or pulleys or gears, utilizing a mechanical intercoupler (994) such as a cable, chain, or belt, as depicted in Figure 6J. Referring to Figures 6K-6N, the secondary interfaces (1002) in this embodiment are mechanically driveably coupled to the instrument interface sockets (44) with a motion transfer interface (1003), such as a worm screw interface. The net result is that the instrument interface sockets (44) are driven by the sheath actuation motors (241) in this embodiment.

Referring to Figure 7, one embodiment of an operator console (8) is depicted, which is configured to operate the subject robotic instrument system in various predefined manners, which shall be illustrated as follows utilizing examples of operation configurations and user interface scenarios.

With a control console (8) such as that depicted in Figure 7, and an instrument driver/instrument configuration such as that depicted in Figures 6A-6N, a cardiac ablation scenario may be illustrated as an example of a diagnostic and/or interventional procedure utilizing the operational aspects of the subject invention. Referring to Figure 8, an instrument driver embodiment (16) is depicted having removably coupled to it a sheath instrument (30) and a guide instrument (18). The distal tubular portion of the guide instrument (18) is movably inserted through the working lumen defined by the sheath instrument (30). A cardiac mapping or ablation catheter (881) is inserted through the working lumen defined by the guide instrument (18), with the proximal handle (1004) of the conventional ablation catheter (881) coupled to a portion of the guide instrument interface (40) to prevent stress from such handle being applied to the guide instrument (18).

Referring to Figure 9, one of the displays (1011) of the operator workstation (element 2 of Figure 2), such as a flatscreen computer monitor, is depicted having a main display field (1005), two auxiliary fields (1006, 1007), a sheath instrument control dashboard (1012), instrument-to-body spatial orientation indicator (1008), a fluoroscopy to body spatial orientation indicator (1009), and load sensing indicator scale (1010) - all of which are presented graphically as graphical user interface objects. The sheath instrument control dashboard may comprise, for example, an indicator of instrument driver (and therefore sheath instrument and guide instrument together in the illustrated embodiments) roll, or "torque", position or instrument driver roll relative to the physical roll limits of the system, sheath instrument distal bend actuation relative to the safe limits of the system or instrument, sheath instrument proximal bend actuation relative to the safe limits of the system or instrument, and sheath instrument insert actuation, by virtue of inserting the illustrated sheath block (185), relative to the safe limits of the system or instrument. Each of these graphical gauge indicators may also be presented with colored indications of safe zones (similar, for example, to the redline indication on an automobile engine tachometer, with the exception that operation out of the safe zones of the instrument preferably is not configured to damage the system - but, in one embodiment, be more difficult to achieve given the limitations of the system). The depicted graphical user interface load sensing indicator may be configured for maximum scale and graduation between zero load and such maximum. The operator console (8) buttons for "intellisense" (826) may be utilized to active load sensing; the button for "baseline" (828) is configured to capture what is believed by the operator to be a baseline (approximately zero) load for load comparison purposes; zoom (830), still (834), clip (836), and review (838) buttons may be utilized to cause the system to magnify or demagnify a depicted image, to capture a still image that is depicted, to capture a movie clip of images being depicted, and to review movies or clips, respectively. A mouse interface comprising, for example, a trackball (824) and two buttons (822) may be utilized for interaction of the operator and system.

Referring to Figure 10A, registration of a sheath/guide/ablation catheter complex to actual fluoroscopy-based images presented in the background in two dimensions may be achieved by depressing the register button (832) on the operator console (8) and using the mouse (822, 824) to place three graphic markers (1020) down the length of the fluorographic images of the sheath instrument (1013), guide instrument (1014), and working instrument (1015). To register in three dimensions, the fluoroscopy imaging plane is changed, generally by moving the swing arm of the fluoroscopy system, and inputting information regarding the new fluoroscopy position to the robotic instrument control system (may be automatic by virtue of an integrated fluoroscopy arm inclinometer, in one embodiment), and an additional three markers (1025) are selected using the mouse (822, 824) - one at the distal tip fluoroscopy image depiction of the guide instrument (1014), and two other markers where projection lines (1023m 1024), based upon the positions of the points previously selected in the other fluoroscopy plane, intersect the second fluorographic image in the background. With the six points (1020, 1025) and relative positions of the fluoroscopy planes determined, the fluoroscopy image may be registered to the "cartoon" images (1016, 1017, 1018) of the instruments displayed based upon a computed position of where the instruments should be (based, for example, upon kinematic calculations).

Also depicted in Figure 10A, longitudinal graduation lines (1022) may be graphically presented upon the cartoon image (1017) of the guide instrument as a method of providing the operator with an indication regarding how much guide instrument has been inserted out of the sheath instrument. Each graduation may, for example, represent an additional centimeter of insertion length. Additionally, transparent shadowing (1021) may be presented to the operator as part of the sheath instrument, guide instrument, or working instrument cartoon presentations (1016, 1017, 1018) as a method of providing the operator with an indication that the pertinent instrument is being directed out of plane from the plane of the monitor. Further, as a method of providing the operator with an indication of which instrument is being actively driven at a given instant, the cartoon presentation (1016, 1017, 1018) of such instrument may be highlighted with a switch of coloration on the displayed cartoon of the instrument; for example, when the guide instrument is inactive, the guide instrument cartoon (1017) may be presented as a generally blue-colored object - but when the guide instrument is being actively driven by the operator, the cartoon depiction of the guide instrument (1017) may be presented as a bright red or salmon-colored object, for example, or may be presented discontinuously to provide a blinking presentation. Similarly, the sheath instrument cartoon presentation (1016) may be highlighted as a method to present the operator with feedback that such instrument is being actively driven with the sheath instrument controls. Further, as a method of providing the operator with feedback that either instrument is in a position of heightened column strength (i.e., when either instrument is in a straight-ahead position, as opposed to a very bent position where bending is more likely than straight column stress when one of the instruments is inserted toward an object such as a tissue structure; for example, when the entire length, or an another embodiment only the distal portion, of either instrument occupies a position within 5 or 10 degrees of straight position), such instrument cartoon presentation may be highlighted with a different readily distinguishable color, such as bright yellow.

Referring to Figure 11, subsequent to registration, the instrument system may be navigated around utilizing the cartoon presentations of the instruments (1016, 1017, 1018) depicted relative to a tissue structure (1026), such as a wall of the right atrium, and some points may be marked with graphical markers (1027) and the coordinates of such markers saved into a database - using the mark point button (840) on the console (8). Each marked point may be, for example, depicted as a colored sphere and may be labeled automatically or manually. Later, these points may be automatically returned to utilizing the electromechanical driveability of the subject robotic instrument system - in a selected order, with a program of events (for example, pauses of time may be selected at each marker location for the working instrument to remain in contact there - or pauses of time with ablation energy applied simultaneously, EKG, impedance, tissue compliance, or other data acquired during such pauses).

Referring to Figure 12, a sequence of operations is illustrated. Subsequent to registration (1028) of the pertinent instruments, such instruments may be accurately navigated toward tissue structures of interest and points of interest utilizing both the fluoroscopy (1013, 1014, 1015) and graphical user interface cartoon (1016, 1017, 1018) depictions of the instruments, and points of interest, such as geometric locations and/or presumed or confirmed points of contact between the instruments and nearby tissue structures or other instruments (such contact determined using, for example, impedance monitoring, force or contact sensing, EKG signal analysis, etc) may be marked and stored by the system. Using the operator workstation (2) interfaces (such as the touchscreen interface (5)), these points may be re-ordered, labeled, color coded, or utilized by a function train which may be programmed by the operator to, for example, have the instruments place the most distal instrument tip at each point for a period of time, heat or ablate during such pauses, monitor EKG, etc, as described above. After the function train is programmed, it may be executed as the operator watches the instruments automatically and electromechanically move through the function train. Such a method may be used, for example, in cardiac mapping, cardiac ablation, or tissue injection therapy scenarios.

As described above, a zoom toggle switch (830) on the depicted embodiment of the operator console (8) may be utilized to zoom in or out on the depicted graphical interface. A still button (834) may be used to capture a screen shot or image of the main display field. A clip button (836) may be utilized to capture a digital video of a particular display field for a selectable period of time. A review button (838) may be utilized to review still shots or video clips. An ICE button (842) may be utilized to switch to a viewing mode comprising an intravascular ultrasound image in addition to instrumentation depictions, as described above. A fluoro view button may be selected (844) to display a fluoroscopy view in addition to instrument depictions, as described above. A "3-d mode" button may be selected (846) to allow one of the displayed user interface fields (1005, 1006, 1007, for example) to be manipulated around in three dimensions using an input such as the mouse (824, 822) or the master input device (12), and depicting selected points. A small graphical user interface presentation of a patient-to-instrument spatial orientation indicator may be configured to rotate around also to depict the position of the depicted instruments relative to the patient on the operating table.

Referring back to Figure 7, several sheath instrument controls are featured on the depicted operator console (8) embodiment, including controls for a special sheath "bend mode" (850), a special "working instrument tip mode" (852), instrument driver (and therefore the instruments also, in the aforementioned depicted embodiments) roll, or "torque", actuation (854), sheath distal bend actuation (858), sheath proximal bend actuation (860), sheath insertion actuation (856), a special "sheath follow the guide" or "sheath follow" mode (862), and a special "guide instrument autoretract" mode (1068).

Referring to Figure 13, the sheath instrument may be inserted or retracted relative to the position of the guide instrument using the "insert" rocker switch (856) on the console (8). Simple sheath insertion absent other consideration may cause the position of the distal portion of the guide to move in space as the sheath instrument creeps up and "swallows" the distal portion of the guide instrument - so if an operator desires to insert the sheath relative to the guide while automatically maintaining the position of the distal tip of the guide, a "sheath follow" rocker switch (862) may be used - which is configured to cause the system to integrate movements of the sheath and guide instruments and result in the sheath "following" along the previous position of the guide, without substantially altering such preexisting guide position. It may be desirable, for example, to use the follow mode to pass the sheath through a transseptal puncture, through which the guide has already passed, to accomplish a substantially minimally invasive a sheath instrument crossing, without additional loads being applied to the tissue puncture location by virtue of the guide instrument and/or sheath instrument moving around as the sheath is advanced over the guide.

Referring to Figure 14, with dual-actuation sheath instrument having one tension element terminating at a more proximal location (1034) and the other tension element terminating on a substantially opposite site of the sheath instrument at the distal tip of the sheath instrument (1035), as described in reference to the illustrated instrument driver (16) embodiments, a distal sheath bend rocker switch (858) and proximal sheath bend rocker switch (860) may be utilized to adjust the position of the sheath instrument - separately or simultaneously.

Referring to Figure 15, an "autoretract" button (1068) is configured to retract the guide instrument along the path that it previously occupied; the distal centerpoint (1038) of the guide instrument is retracted along the path formed by longitudinal centerpoints (1037) previously occupied by more proximal portions of the guide instrument and/or sheath instrument. This feature may be utilized to safely withdraw the guide instrument in various scenarios when minimizing interference with nearby structures is important (and the operator knows that the guide instrument previously occupied the pathway - so the guide instrument presumably may be pulled back along such pathway safely).

Referring to Figure 16, a sheath "bend" mode may be utilized (by pressing the bend button (850)) to lock the position of the distal sheath, which may be presented as a graphical user interface marker (1039), in space while adjusting the shape of the proximal sheath instrument - to modify the trajectory of the sheath and/or guide, for example. Referring to Figure 17, an instrument "tip" mode (actuated by pressing the tip button (852)) similarly locks the position of the distal guide, or in another embodiment the position of the distal tip of the working instrument, in space to allow for adjustment of the sheath. The tip position being locked may be depicted as a graphical user interface marker (1040), as shown in Figure 17. Referring to Figure 18, both sheath and bend modes may be actuated simultaneously to lock the distal tips of both the sheath and the guide in position while the more proximal shape of the sheath is adjusted.

Referring to Figure 19, one embodiment of a configuration for operating the subject system of steerable instruments is depicted. After the instruments have been registered (1028), they may be navigated with precision toward tissue structures of interest (1029). For example, in an atrial fibrillation application, they may be navigated from the inferior vena cava, across the right atrium, toward the fossa ovalis of the septum, a common target for trans-septal puncture. Subsequent to reaching the desired tissue structure, before advancing a tool out of the working lumen of the guide instrument, it may be desirable to reposition or optimize the shape of the sheath instrument (1041). For example, in the trans-septal scenario, before advancing a trans-septal needle, it may be desirable to carefully tune the trajectory of the needle to avoid important adjacent tissue structures, such as the aortic outflow tract. The sheath positioning (distal bend, proximal bend, insertion) functions, as well as the "follow" function (wherein the aforementioned functions may be integrated to follow along the trajectory of the guide instrument without repositioning the distal guide instrument) may be utilized for such objectives, as well as the guide instrument navigation via the master input device (12).

After optimizing the position and shape of the pertinent instruments, the tool may be advanced into the targeted tissue structure with precision trajectory and location (1042). Should it be desirable to cross the targeted tissue structure, for example in a trans-septal scenario, the guide instrument may be advanced over the tool (1043) and the sheath subsequently over the guide (for example, using the "follow" mode described above) to position the distal tips of both instruments across the targeted tissue structure (1044). From there, the distal tip of the sheath may be locked into position (1045) utilizing the "bend" mode desribed above (or the "bend" + "tip" mode to also lock the tip of the guide instrument in place, perhaps during sheath instrument reshaping to provide better trajectory for the guide instrument, in one embodiment to alter the position of the guide instrument workspace within the left atrium to provide access to desired tissue structures), and the guide navigated (1046) forward from there (in one embodiment carrying, for example, an ablation catheter or other tool to mark points or create lesions - 1047).

Referring to Figure 20, a configuration for utilizing a working instrument force sensing functionality is depicted. A registered instrument or set of instruments may be navigated toward a tissue structure of interest with force sensing activated (1048), as described, for example, in U.S. Patent Application Serial No. 11/678,016, filed February 22, 2007. Once the system provides feedback (preferably via the force sensing scale adjacent the main display field, as depicted in Figure 9) that the instruments are in contact with something, such contact may be confirmed with other indicators (1049 - for example, EKG signal, mismatch between localized position and computed position - or between computed position and fluoroscopy position, impedance monitoring, etc). Subsequently, to gain an precision force sensing signal given the position and shape of the pertinent steerable instruments, the instruments may be retracted away, for example, using the autoretract functionality, into a position of free space (1053) wherein lack of contact can be confirmed by zero load indicated from the force sensor, in addition to other indicators (lack of EKG signal, close match between localized position and computed position - or between fluoro position and computed position, impedance monitoring, etc), and the force sensing system may be baselined (1057). Subsequently the tissue structure may be reapproached and force sensed (1059) with enhanced accuracy given the recent baselining with a similar instrument shape factor and position, and points may be marked, lesions created, etc. (1061).

To reduce overcompression of the guide instrument while also preventing slack of tension elements which may be associated with loss of steering control, a net load, such as 8 pounds, may be maintained in the tension elements (for example, 2 pounds in each of 4 tension elements to start) - then while the net compressive load on the body of the guide instrument is maintained at this net amount, the loads relative to each other of the individual tension elements may be decreased or increased to induce bending/steering of the guide instrument.

Referring to Figure 21, in one embodiment, localization sensing may be utilized to assist in error detection and contact sensing interpretation. As shown in Figure 21, an instrument system variation comprising a sheath instrument, guide instrument, ablation catheter (or other instrument so located; an ablation instrument presents a simple instrument for illustration purposes), and localization sensor (shown in two optional locations - one wherein the sensor comprises a portion of the distal tip of the ablation catheter - 1062, one wherein the sensor comprises a portion of the distal tip of the guide instrument - 1063; many other locations are suitable). The system may be configured to display a spherical colored marker (semi-transparent, for example - illustration elements 1065, 1066) about either the center of the distal tip of the guide instrument or the center of the distal tip of the ablation catheter using two sources of information regarding where such center of distal instrument location is located: 1) the computed located based upon inverse kinematics and instrument mechanics, and 2) the located based upon feedback from the localization system. If the localization system is known to be accurate, and the inverse-kinematics-based calculation is accurate in free space, then the two spherical markers (1065, 1066) should be substantially aligned in space. When the two spherical markers are not aligned, this may be interpreted as error in either system, or an external factor, such as contact with a tissue structure, which is preventing the localized position (assumed, in such example, to be a more accurate representation of reality) from reaching the position that the system believes the instrument has reached based upon inverse kinematics and associated servomotor currents (an indication of tension element load, for example). In such embodiment, a line (1066) may be graphically presented between the centers of the two depicted spherical markers - so the operator may interpret the length of such line as error in one of the systems, or contact with an external factor, such as a tissue structure.

While multiple embodiments and variations of the many aspects of the invention have been disclosed and described herein, such disclosure is provided for purposes of illustration only. Many combinations and permutations of the disclosed system are useful in minimally invasive surgery, and the system is configured to be flexible. For example, depending upon the medical application, it may be desirable to have a guide instrument with less than four control elements, combined with a sheath instrument, or perhaps combined with a prebent, unsteerable sheath, or perhaps with no sheath at all. The instrument driver may be tailored to match the instrument configuration, with less motors and gearboxes for less control elements, or variation in the configuration for actuating a given control element interface assembly, and associated variation in the tensioning mechanism and number of control element pulleys associated with the pertinent control element interface assembly (one pulley and one cable per control element interface assembly, two pulleys and two cables per control element interface assembly, slotted, split carriage, and winged split carriage embodiments, various tensioning embodiments, etc).

## Claims

1. A robotic instrument system, comprising:
a controller (6) including a master input device (12) and an operator control console (8); and
an instrument driver (16) in communication with the controller (6), the instrument driver comprising
a guide instrument interface (18) including two or more guide instrument drive elements responsive to control signals generated, at least in part, by the master input device (12) for manipulating a guide instrument (1014) operatively coupled to the instrument interface (18), and
a sheath instrument interface (30) including two or more sheath instrument drive elements responsive to control signals generated, at least in part, by the master input device (12) for manipulating a sheath instrument (1013) operatively coupled to the instrument interface (30), wherein the sheath instrument drive elements are controlled independently of the guide instrument drive elements, and wherein
the operator control console (8) comprises a sheath bend mode control (850) for locking the position of the distal sheath instrument in space while adjusting the shape of the proximal sheath instrument.

2. A robotic instrument system, comprising:
a controller (6) including a master input device (12) and an operator control console (8); and
an instrument driver (16) in communication with the controller (6), the instrument driver comprising
a guide instrument interface (18) including two or more guide instrument drive elements responsive to control signals generated, at least in part, by the master input device (12) for manipulating a guide instrument (1014) operatively coupled to the instrument interface (18), and
a sheath instrument interface (30) including two or more sheath instrument drive elements responsive to control signals generated, at least in part, by the master input device (12) for manipulating a sheath instrument (1013) operatively coupled to the instrument interface (30), wherein the sheath instrument drive elements are controlled independently of the guide instrument drive elements, and wherein the operator control console (8) comprises an instrument tip mode control (852) for locking the position of the distal guide, or the position of the distal tip of a working instrument (1015), in space to allow for adjustment of the sheath instrument (1013).

3. The system of claim 1, wherein the operator control console (8) comprises in addition an instrument tip mode control (852) for locking the position of the distal guide, or the position of the distal tip of a working instrument (1015), in space to allow for adjustment of the sheath instrument (1013), and wherein both sheath bend and instrument tip modes (850, 852) can be actuated simultaneously to lock the distal tips of both the sheath instrument (1013) and the guide instrument (1014) in position while the more proximal shape of the sheath instrument (1013) can be adjusted.

4. The system of any one of claims 1 to 3, wherein each of the two or more guide instrument drive elements are controlled independently of the other guide instrument drive elements.

5. The system of any one of claims 1 to 4, wherein each of the two or more sheath instrument drive elements are controlled independently of the other sheath instrument drive elements.

6. The system of any one of claims 1-5, wherein the respective guide instrument interface (18) and sheath instrument interface (30) are positioned on the instrument driver (16) such that a portion of a guide instrument (1014) operatively coupled to the guide instrument interface (18) is disposed coaxially within a lumen of a sheath instrument (1013) operatively coupled to the sheath instrument interface (30).

7. The system of any one of claims 1-6, wherein the guide instrument interface (18) is controllably movable along a guide instrument insertion trajectory, and the sheath instrument interface (30) is controllably movable along a sheath instrument insertion trajectory independently of movement of the guide instrument interface.

8. The system of claim 7, wherein the guide instrument trajectory is substantially aligned with the sheath instrument trajectory.

9. The system of claim 7, wherein movement of the guide instrument interface (18) is accomplished by controlled rotation of a respective guide insertion lead screw.

10. The system of any of claim 7, wherein movement of the sheath instrument interface (30) is accomplished by controlled rotation of a respective sheath insertion lead screw.

11. The system of claim 7, wherein movement of the guide instrument interface is accomplished by a respective guide insertion cable and pulley assembly.

12. The system of claim 7, wherein movement of the sheath instrument interface is accomplished by a respective sheath insertion cable and pulley assembly.

## Patentansprüche

1. Roboterinstrumentensystem, umfassend:
ein Steuergerät (6), das eine Haupteingabeeinrichtung (12) und eine Bedienperson-Steuerkonsole (8) beinhaltet; und
einen Instrumentenantrieb (16), der mit dem Steuergerät (6) in Verbindung steht, wobei der Instrumentenantrieb umfasst
eine Führungsinstrumentenschnittstelle (18), die zwei oder mehr Führungsinstrument-Antriebselemente beinhaltet, die auf Steuersignale reagieren, die mindestens teilweise von der Haupteingabevorrichtung (12) generiert werden, um ein wirksam mit der Führungsinstrumentenschnittstelle (18) gekoppeltes Führungsinstrument (1014) zu manipulieren, und
eine Hülseninstrumentenschnittstelle (30), die zwei oder mehr Hülseninstrument-Antriebselemente beinhaltet, die auf Steuersignale reagieren, die mindestens teilweise von der Haupteingabevorrichtung (12) generiert werden, um ein wirksam mit der Instrumentenschnittstelle (30) gekoppeltes Hülseninstrument (1013) zu manipulieren, wobei die Hülseninstrument-Antriebselemente unabhängig von den Führungsinstrument-Antriebselementen gesteuert werden, und wobei
die Bedienperson-Steuerkonsole (8) eine Hülsenbiegungsmodus-Steuerung (850) zum Verriegeln der Position des distalen Hülseninstruments im Raum, während die Form des proximalen Hülseninstruments angepasst wird, umfasst.

2. Roboterinstrumentensystem, umfassend:
ein Steuergerät (6), das eine Haupteingabevorrichtung (12) und eine Bedienperson-Steuerkonsole (8) beinhaltet; und
einen Instrumentenantrieb (16), der mit dem Steuergerät (6) in Verbindung steht, wobei der Instrumentenantrieb umfasst
eine Führungsinstrumentenschnittstelle (18), die zwei oder mehr Führungsinstrument-Antriebselemente beinhaltet, die auf Steuersignale reagieren, die mindestens teilweise von der Haupteingabevorrichtung (12) generiert werden, um ein wirksam mit der Führungsinstrumentenschnittstelle (18) gekoppeltes Führungsinstrument (1014) zu manipulieren, und
eine Hülseninstrumentenschnittstelle (30), die zwei oder mehr Hülseninstrument-Antriebselemente beinhaltet, die auf Steuersignale reagieren, die mindestens teilweise von der Haupteingabevorrichtung (12) generiert werden, um ein wirksam mit der Instrumentenschnittstelle (30) gekoppeltes Hülseninstrument (1013) zu manipulieren, wobei die Hülseninstrument-Antriebselemente unabhängig von den Führungsinstrument-Antriebselementen gesteuert werden, und wobei
die Bedienperson-Steuerkonsole (8) eine Instrumentenspitzenmodus-Steuerung (852) zum Verriegeln der Position der distalen Führung, oder der Position der distalen Spitze eines Arbeitsinstruments (1015), im Raum umfasst, um ein Anpassen des Hülseninstruments (1013) zu gestatten.

3. System nach Anspruch 1, wobei die Bedienperson-Steuerkonsole (8) zusätzlich eine Instrumentenspitzenmodus-Steuerung (852) zum Verriegeln der Position der distalen Führung, oder der Position der distalen Spitze eines Arbeitsinstruments (1015), im Raum umfasst, um ein Anpassen des Hülseninstruments (1013) zu gestatten, und wobei sowohl Hülsenbiegungs- als auch Instrumentenspitzenmodus (850, 852) gleichzeitig betätigt werden können, um die distalen Spitzen sowohl des Hülseninstruments (1013) als auch des Führungsinstruments (1014) in ihrer Position zu verriegeln, während die proximalere Form des Hülseninstruments (1013) angepasst werden kann.

4. System nach einem der Ansprüche 1 bis 3, wobei jedes der zwei oder mehr Führungsinstrument-Antriebselemente unabhängig von den anderen Führungsinstrument-Antriebselementen gesteuert wird.

5. System nach einem der Ansprüche 1 bis 4, wobei jedes der zwei oder mehr Hülseninstrument-Antriebselemente unabhängig von den anderen Hülseninstrument-Antriebselementen gesteuert wird.

6. System nach einem der Ansprüche 1-5, wobei die jeweilige Führungsinstrumentenschnittstelle (18) und Hülseninstrumentenschnittstelle (30) so an dem Instrumentenantrieb (16) positioniert sind, dass ein Teil eines wirksam an die Führungsinstrumentenschnittstelle (18) gekoppelten Führungsinstruments (1014) koaxial in einem Lumen eines wirksam an die Hülseninstrumentenschnittstelle (30) gekoppelten Hülseninstruments (1013) angeordnet ist.

7. System nach einem der Ansprüche 1-6, wobei die Führungsinstrumentenschnittstelle (18) steuerbar entlang einer Führungsinstrumenten-Einbringbahn bewegbar ist und die Hülseninstrumentenschnittstelle (30) steuerbar entlang einer Hülseninstrumenten-Einbringbahn bewegbar ist, unabhängig von der Bewegung der Führungsinstrumentenschnittstelle.

8. System nach Anspruch 7, wobei die Führungsinstrumentenbahn im Wesentlichen auf einer Linie mit der Hülseninstrumentenbahn liegt.

9. System nach Anspruch 7, wobei die Bewegung der Führungsinstrumentenschnittstelle (18) durch gesteuerte Rotation einer jeweiligen Führungseinbring-Leitspindel vollzogen wird.

10. System nach einem von Anspruch 7, wobei die Bewegung der Hülseninstrumentenschnittstelle (30) durch gesteuerte Rotation einer jeweiligen Hülseneinbring-Leitspindel vollzogen wird.

11. System nach Anspruch 7, wobei die Bewegung der Führungsinstrumentenschnittstelle durch eine jeweilige Anordnung aus Führungseinbring-Seilzug und Seilrolle vollzogen wird.

12. System nach Anspruch 7, wobei die Bewegung der Hülseninstrumentenschnittstelle durch eine jeweilige Anordnung aus Hülseneinbring-Seilzug und Seilrolle vollzogen wird.

## Revendications

1. Système robotique d'instrument comprenant :
un contrôleur (6) englobantun dispositif d'entrée maître (12) et une console de commande d'opérateur(8) ; et
un pilote d'instrument (16) mis en communication avec le contrôleur (6), le pilote d'instrument comprenant :
une interface d'instrument faisant office de guide (18) englobant deux éléments d'entraînement d'instrument faisant office de guide ou plus sensibles à des signaux de commande générés au moins en partie parle dispositif d'entrée maître (12) pour manipuler un instrument faisant office de guide (1014) couplé de manière opérante à l'interface d'instrument (18) ;
- une interface d'instrument faisant office de gaine (30) englobant deux éléments d'entraînement d'instrument en forme de gaine ou plus sensibles à des signaux de commande générés au moins en partie parle dispositif d'entrée maître (12) pour manipuler un instrument faisant office de gaine (1013) couplé de manière opérante à l'interface d'instrument (30), les éléments d'entraînement d'instruments de gaine étant commandés indépendamment des éléments d'entraînement d'instruments faisant office de guide et la console de commande d'opérateur (8) comprenant une commande en mode de flexion de gaine (850) pour bloquer la position de l'instrument distal faisant office de gaine dans l'espace tandis que l'on règle la configuration de l'instrument proximal faisant office de gaine.

2. Système robotique d'instrument comprenant :
un contrôleur (6) englobantun dispositif d'entrée maître (12) et une console de commande d'opérateur(8) ; et
un pilote d'instrument (16) mis en communication avec le contrôleur(6), le pilote d'instrument comprenant :
une interface d'instrument faisant office de guide (18) englobant deux éléments d'entraînement d'instrument faisant office de guide ou plus sensibles à des signaux de commande générés au moins en partie parle dispositif d'entrée maître (12) pour manipuler un instrument faisant office de guide (1014) couplé de manière opérante à l'interface d'instrument (18) ;
- une interface d'instrument faisant office de gaine (30) englobant deux éléments d'entraînement d'instrument en forme de gaine ou plus sensibles à des signaux de commande générés au moins en partie parle dispositif d'entrée maître (12) pour manipuler un instrument faisant office de gaine (1013) couplé de manière opérante à l'interface d'instrument (30), les éléments d'entraînement d'instruments de gaine étant commandés de manière indépendante par rapport aux éléments d'entraînement d'instrument faisant office de guide et la console de commande d'opérateur(8) comprenant une commande en mode de pointe d'instrument (852) pour bloquer la position du guide distal ou la position de la pointe distale d'un instrument de travail (1015) dans l'espace pour permettre le réglage de l'instrument faisant office de gaine (1013).

3. Système selon la revendication 1, dans lequel la console de commande d'opérateur (8) comprend en outre une commande en mode de pointe d'instrument (852) pour bloquerla position du guide distal ou la position de la pointe distale d'un instrument de travail (1015) dans l'espace pour permettre le réglage de l'instrument faisant office de gaine (1013), et dans lequel à la fois le mode en flexion de gaine et le mode de pointe d'instrument (850, 852) peuvent être mis en oeuvre de manière simultanée pour bloquer les extrémités distalesà la fois de l'instrument faisant office de gaine (1013) et de l'instrument faisant office de guide (1014) en position, tandis que l'on peut régler la configuration plus proximale de l'instrument faisant office de gaine (1013).

4. Système selon l'une quelconque des revendications 1 à 3, dans lequel chacun des deux éléments d'entraînement d'instruments faisant office de guide ou plus sont commandés indépendamment des autres éléments d'entraînement d'instruments faisant office de guide.

5. Système selon l'une quelconque des revendications 1 à 4, dans lequel chacun des deux éléments d'entraînement d'instruments faisant office de gaine ou plus sont commandés indépendamment des autres éléments d'entraînement d'instruments faisant office de gaine.

6. Système selon l'une quelconque des revendications 1 à 5, dans lequel l'interface d'instrument faisant office de guide (18) et l'interface d'instrument faisant office de gaine (30) sont disposées respectivement sur le pilote d'instrument (16) de telle sorte qu'une portion d'un instrument faisant office de guide (1014) couplée de manière opérante à l'interface d'instrument faisant office de guide (18) est disposée en position coaxiale au sein d'une lumière d'un instrument faisant office de gaine (1013) couplé de manière opérante à l'interface d'instrument faisant office de gaine (30).

7. Système selon l'une quelconque des revendications 1 à 6, dans lequel l'interface d'instrument faisant office de guide (18) peut se déplacer de manière réglable le long d'une trajectoire d'insertion d'instrument faisant office de guide, et l'interface d'instrument faisant office de gaine (30) peut se déplacer de manière réglable le long d'une trajectoire d'insertion d'instrument faisant office de gaine, indépendamment du mouvementde l'interface d'instrument faisant office de guide.

8. Système selon la revendication 7, dans lequel la trajectoire d'instrument faisant office de guide est essentiellement mise en alignement avec la trajectoire d'instrument faisant office de gaine.

9. Système selon la revendication 7, dans lequel le mouvement de l'interface d'instrument faisant office de guide (18) est réalisé par rotation contrôlée d'une vis mère respective d'insertion de guide.

10. Système selon la revendication 7, dans lequel le mouvement de l'interface d'instrument faisant office de gaine (30) est réalisé par rotation contrôlée d'une vis mère respective d'insertion de gaine.

11. Système selon la revendication 7, dans lequel le mouvement de l'interface d'instrument faisant office de guide est réalisé parun assemblage respectif de câble et de poulie d'insertion de guide.

12. Système selon la revendication 7, dans lequel le mouvement de l'interface d'instrument faisant office de gaine est réalisé parun assemblage respectif de câble et de poulie d'insertion de gaine.
